# FASCICULE DE BREVET EUROPEEN

(11) **EP 1 633 421 B1**
(45) Date de publication et mention de la délivrance du brevet: **04.03.2020**
(21) Numéro de dépôt: 04742738.0
(22) Date de dépôt: 14.05.2004
(51) Int. Cl.: A61M 15/00

(54) **DISTRIBUTEUR DE PRODUIT FLUIDE**
FLUIDPRODUKTSPENDER
FLUID PRODUCT DISPENSER

(30) Priorité: 15.05.2003 FR 0305858
(43) Date de publication de la demande: 15.03.2006
(73) Titulaire: Aptar France SAS, 27110 Le Neubourg (FR)
(72) Inventeur: POULARD, Fabien, F-76000 Rouen (FR)
(74) Mandataire: CAPRI
(86) Numéro de dépôt international: PCT/FR2004/001189
(87) Numéro de publication internationale: WO 2004/101042

(56) Documents cités:
- EP-A- 0 480 488
- DE-A- 10 061 723
- US-A1- 2002 195 102
- US-B1- 6 446 627

## Description

La présente invention concerne un distributeur de produit fluide, et plus particulièrement un tel distributeur comportant un dispositif d'indication de doses pour indiquer à l'utilisateur le nombre de doses distribuées ou restant à distribuer à partir du réservoir dudit distributeur de produit fluide.

Les dispositifs d'indication de doses sont bien connus, et peuvent comporter soit des compteurs, affichant un chiffre correspondant au nombre de doses distribuées ou restant à distribuer, soit des indicateurs, informant l'utilisateur par des symboles, codes couleurs, chiffres similaire sur le nombre de doses distribuées ou restant à distribuer. En particulier dans les distributeurs de produit fluide contenant des produits pharmaceutiques, il est important que le dispositif d'indication de doses fonctionne de manière fiable, et notamment qu'il compte la distribution d'une dose à chaque fois que du produit est distribuée, que la dose soit complète ou partielle, par exemple en raison d'un actionnement défaillant ou interrompu avant la fin du cycle d'actionnement. En effet, il est généralement préférable de compter la distribution d'une dose incomplète, plutôt que de ne pas la compter, ce qui peut présenter un risque important vis-à-vis de l'utilisateur en lui indiquant un contenu du réservoir supérieur à la réalité. Dans les distributeurs de produit pharmaceutique, on souhaite donc généralement éviter tout risque de sous-comptage, notamment en permettant le comptage juste avant l'expulsion de produit actif. Un autre point important avec les dispositifs d'indication de doses de distributeur de produits pharmaceutiques, est qu'après un actionnement et une distribution d'une dose, lorsque le distributeur revient dans sa position de repos, tout nouvel actionnement réalisé avant la fin de la course de retour du distributeur et qui occasionnerait la distribution d'une dose complète ou partielle, devrait également être compté par le dispositif d'indication, pour à nouveau éviter tout risque de sous-comptage. Dans la plupart des distributeurs de produit fluide, après distribution d'une dose, la prochaine dose est chargée dans la chambre de l'organe de distribution (pompe ou valve) lors de la course de retour du distributeur vers sa position de repos. Pour éviter tout risque de sous-comptage lors de la course de retour du distributeur, il est souhaitable que le distributeur de produit fluide soit bloqué à partir du moment où la course de retour a permis le remplissage de la chambre et jusqu'à ce que le dispositif d'indication soit de nouveau apte à compter l'actionnement du distributeur. Le document US2002/0195102 décrit un compteur de l'art antérieur.

La présente invention a pour but de fournir un distributeur de produit fluide qui remplit la ou les exigences susmentionnées.

En particulier, la présente invention a pour but de fournir un distributeur de produit fluide comportant un dispositif d'indication de doses, qui évite tout risque de sous-comptage, c'est-à-dire qui garantisse l'actionnement du dispositif d'indication à chaque fois que du produit fluide est distribué par le distributeur de produit fluide.

La présente invention a également pour but de fournir un tel distributeur, qui évite de compter la distribution d'une dose, et donc d'actionner le dispositif d'indication de doses lorsque l'actionnement du distributeur n'implique aucune distribution de produit fluide.

La présente invention a encore pour but de fournir un tel distributeur de produit fluide qui soit simple et peu coûteux à fabriquer et assembler, et fiable d'utilisation.

La présente invention a donc pour objet un distributeur de produit fluide, comportant un corps, un réservoir de produit fluide, un organe de distribution, tel qu'une pompe ou une valve, monté sur ledit réservoir, et un dispositif d'indication de doses pour indiquer le nombre de doses de produit distribuées ou restant à distribuer à partir dudit réservoir, caractérisé en ce que ledit dispositif d'indication de doses comporte un premier système de sécurité destiné à actionner le dispositif d'indication de doses à partir d'une course d'actionnement partielle prédéterminée du distributeur, même si la course d'actionnement totale n'est pas réalisée par le distributeur.

De préférence, ledit dispositif d'indication de doses comporte un second système de sécurité qui, lors de la course retour du distributeur, après distribution d'une dose, empêche la prochaine expulsion de produit pharmaceutique jusqu'à ce que ledit distributeur a accompli une course de retour partielle prédéterminée, ledit distributeur et ledit dispositif d'indication de doses pouvant à nouveau être actionnés à partir de cette course de retour partielle prédéterminée, même si la course de retour totale n'est pas réalisée par le distributeur et que celui-ci est actionné à nouveau avant de revenir à sa position de repos.

Avantageusement, ledit réservoir est déplaçable axialement par rapport audit corps, ledit corps comportant au moins un engrenage fixe, ledit dispositif d'indication de doses comportant un élément de comptage déplaçable axialement et en rotation par rapport audit corps, ledit élément de comptage coopérant d'une part avec ledit au moins un engrenage fixe dudit corps et d'autre part avec ledit réservoir lorsque le distributeur est actionné.

Selon un premier mode de réalisation de l'invention, ledit corps comporte un engrenage fixe coopérant avec un premier engrenage dudit élément de comptage, ledit élément de comptage comportant un second engrenage coopérant avec un engrenage d'actionnement d'un organe d'actionnement du distributeur, les dents dudit second engrenage et/ou dudit engrenage d'actionnement étant réalisées de telle sorte qu'un déplacement axial de l'organe d'actionnement sollicite ledit élément de comptage à se déplacer axialement et en rotation, l'engrenage fixe empêchant une rotation dudit élément de comptage jusqu'à ce que ledit élément de comptage ne coopère plus avec ledit engrenage fixe, après un déplacement axial prédéterminé dudit élément de comptage correspondant à ladite course d'actionnement partielle prédéterminée du distributeur.

Avantageusement, l'engrenage fixe comporte des moyens de butée bloquant en rotation ledit élément de comptage après au moins une rotation partielle dudit élément de comptage, un déplacement axial supplémentaire dudit élément de comptage étant nécessaire pour permettre la poursuite de sa rotation et/ou le retour du dispositif d'indication vers sa position de repos. Ces butées positionnent le second engrenage de l'élément de comptage de tel sorte que le distributeur soit bloqué à son retour avant le remplissage de la chambre et jusqu'à sa position de repos.

Avantageusement, lesdits moyens de butée comportent une projection axiale.

Avantageusement, ledit engrenage fixe et/ou ledit élément de comptage comporte(nt) des moyens de blocage empêchant un nouvel actionnement du distributeur, et donc une nouvelle expulsion de produit pharmaceutique, lorsque l'élément de comptage revient vers sa position de repos après un actionnement précédent, jusqu'à ce que le distributeur réalise une course de retour partielle prédéterminée à partir de laquelle le dispositif d'indication peut compter la prochaine dose.

Avantageusement, lesdits moyens de blocage comportent des projections axiales prévues respectivement sur le corps et sur l'élément de comptage, lesdites projections comportent chacune un profil d'extrémité axiale plan, lesdites projections étant au moins partiellement face à face jusqu'à ce que l'élément de comptage a accompli une rotation suffisante pour décaler lesdites projections correspondant à ladite course de retour partielle prédéterminée du distributeur.

Avantageusement, les dents du second engrenage de l'élément de comptage comportant un palier intermédiaire, l'organe d'actionnement coopérant avec ledit palier lors de l'actionnement du distributeur, et coopérant avec le fond dudit second engrenage lors du retour vers la position de repos, après actionnement, le déplacement entre le palier intermédiaire et le fond étant obtenu par rotation dudit élément de comptage.

Avantageusement, lorsque la course de retour partielle prédéterminée a été réalisée, l'organe d'actionnement est placé face à la dent suivante du second engrenage de l'élément de comptage, permettant un nouvel actionnement du distributeur et du dispositif d'indication de doses.

Selon un second mode de réalisation de la présente invention, ledit corps comporte un premier engrenage fixe et un second engrenage fixe, ledit élément de comptage comportant un premier engrenage destiné à coopérer avec ledit premier engrenage fixe, et un second engrenage destiné à coopérer avec ledit second engrenage fixe, ledit élément de comptage étant axialement mis en contact avec l'organe d'actionnement du distributeur par l'intermédiaire d'un ressort de rappel et étant déplaçable en rotation par rapport audit organe d'actionnement, les dents dudit second engrenage et second engrenage fixe étant au moins partiellement obliques de telle sorte qu'un déplacement axial de l'organe d'actionnement sollicite d'abord ledit élément de comptage à se déplacer axialement sur une course d'actionnement partielle prédéterminée, jusqu'à ce que la partie oblique dudit second engrenage de l'élément de comptage coopère avec ladite partie oblique dudit second engrenage fixe, sollicitant l'élément de comptage à se déplacer en rotation sur une première partie de cycle de rotation, les dents desdits premier engrenage et premier engrenage fixe étant au moins partiellement obliques de telle sorte que lorsque l'élément de comptage revient vers sa position de repos, il est sollicité en rotation pour terminer son cycle de rotation correspondant au comptage d'un actionnement du distributeur.

Avantageusement, lesdits premier et second engrenages fixes du corps et/ou lesdits premier et second engrenages de l'élément de comptage sont décalés l'un par rapport à l'autre de telle sorte que dès que l'élément de comptage est déplaçable en rotation, un retour de celui-ci vers sa position de repos sans terminer la course d'actionnement du distributeur entraîne la rotation de l'élément de comptage sur son cycle complet de rotation, garantissant le comptage d'un actionnement du distributeur après ladite course d'actionnement partielle prédéterminée.

Avantageusement, ledit second engrenage fixe du corps et/ou ledit second engrenage de l'élément de comptage comporte(nt) des moyens de blocage empêchant un nouvel actionnement du distributeur, et donc une nouvelle expulsion de produit pharmaceutique lorsque l'élément de comptage revient vers sa position de repos après un actionnement précédent jusqu'à ce que ledit distributeur réalise une course de retour partielle prédéterminée à partir de laquelle le dispositif d'indication peut compter la prochaine dose.

Avantageusement, lesdits moyens de blocage comportent un profil d'extrémité axial au moins partiellement plan formé sur les dents dudit second engrenage fixe du corps et dudit second engrenage de l'élément de comptage, lesdits profils plan desdites dents étant au moins partiellement face à face jusqu'à ce que l'élément de comptage a accompli une rotation suffisante pour décaler lesdites dents, correspondant à ladite course de retour partielle prédéterminée du distributeur.

Avantageusement, ledit organe d'actionnement du distributeur est solidaire dudit réservoir et se déplace axialement avec lui.

Selon un troisième mode de réalisation de la présente invention, ledit corps comporte un premier engrenage fixe et un second engrenage fixe, ledit élément de comptage comportant un premier engrenage destiné à coopérer avec ledit premier engrenage fixe, un second engrenage destiné à coopérer avec ledit second engrenage fixe, et un troisième engrenage destiné à coopérer avec un engrenage d'actionnement, solidaire d'un organe d'actionnement du distributeur, les dents dudit troisième engrenage et/ou dudit engrenage d'actionnement étant réalisées de tel sorte qu'un déplacement axial de l'organe d'actionnement sollicite ledit élément de comptage à se déplacer axialement et en rotation, le premier engrenage fixe empêchant une rotation dudit élément de comptage jusqu'à ce que ledit élément de comptage ne coopère plus avec ledit premier engrenage fixe, après un déplacement axial prédéterminé dudit élément de comptage correspondant à ladite course d'actionnement partielle prédéterminée du distributeur.

Avantageusement, ledit second engrenage fixe du corps et/ou ledit second engrenage de l'élément de comptage comporte(nt) des moyens de blocage empêchant un nouvel actionnement du distributeur, et donc une nouvelle expulsion de produit pharmaceutique lorsque l'élément de comptage revient vers sa position de repos après un actionnement précédent jusqu'à ce que ledit distributeur réalise une course de retour partielle prédéterminée à partir de laquelle le dispositif d'indication peut compter la prochaine dose.

Avantageusement, lesdits moyens de blocage comportent un profil d'extrémité axial plan formé sur les dents dudit second engrenage fixe et dudit second engrenage, lesdites dents étant au moins partiellement face à face jusqu'à ce que l'élément de comptage a accompli une rotation suffisante pour décaler lesdites dents, correspondant à ladite course de retour partielle prédéterminée du distributeur.

Avantageusement, ledit engrenage d'actionnement comporte des moyens de butée limitant la rotation de l'élément de comptage jusqu'à ce que l'organe d'actionnement a réalisé ladite course de retour partielle prédéterminée.

Avantageusement, lesdits moyens de butée comportent une projection axiale formée sur ledit engrenage d'actionnement.

Avantageusement, ledit organe d'actionnement du distributeur est solidaire dudit réservoir et se déplace axialement avec lui.

D'autres caractéristiques et avantages de la présente invention apparaîtront plus clairement au cours de la description détaillée suivante de trois modes de réalisation de celle-ci, faite en référence aux dessins joints, donnés à titre d'exemples non limitatifs, et sur lesquels
- les figures 1 à 8 sont des représentations schématiques d'un distributeur de produit fluide selon un premier mode de réalisation de la présente invention, montrant les positions successives lors d'un cycle d'actionnement du distributeur ;
- les figures 9 à 12 sont des représentations schématiques d'un distributeur selon un second mode de réalisation de la présente invention, montrant également diverses positions dans le cycle d'actionnement du distributeur ; et
- les figures 13 à 21 sont des représentations schématiques d'un distributeur de produit fluide selon un troisième mode de réalisation de la présente invention, montrant différentes positions dans le cycle d'actionnement du distributeur.

Les descriptions des trois modes de réalisation qui vont être faites ci-après concernent les cycles d'actionnement du distributeur et les différentes spécificités fonctionnelles du dispositif d'indication permettant de garantir la fiabilité de fonctionnement et de comptage dudit dispositif d'indication. Les dessins auxquels la présente description fera référence sont donc des schémas très simplifiés, qui ne représentent pas le distributeur de produit fluide en détail, mais seulement de manière très schématisée par les différentes parties déplaçables les une par rapport aux autres, pour permettre l'explication du cycle d'actionnement dudit distributeur et dudit dispositif d'indication. Ainsi par exemple, l'organe de distribution, par exemple une pompe ou une valve, n'est pas représentée sur les dessins. De même, la tête de distribution comportant l'orifice de distribution n'est pas non plus représentée, ces éléments n'intervenant pas directement dans la présente invention.

Il est à noter que la présente invention s'applique plus particulièrement aux dispositifs dits MDI (METERED DOSE INHALER), qui comportent une valve doseuse montée sur un réservoir contenant du produit fluide et un gaz propulseur, le déplacement du réservoir par rapport à la soupape de la valve engendrant la distribution d'une dose de produit au moyen dudit gaz propulseur. La présente invention n'est pas limitée à cette application particulière, mais celle-ci représente l'application préférée de la présente invention.

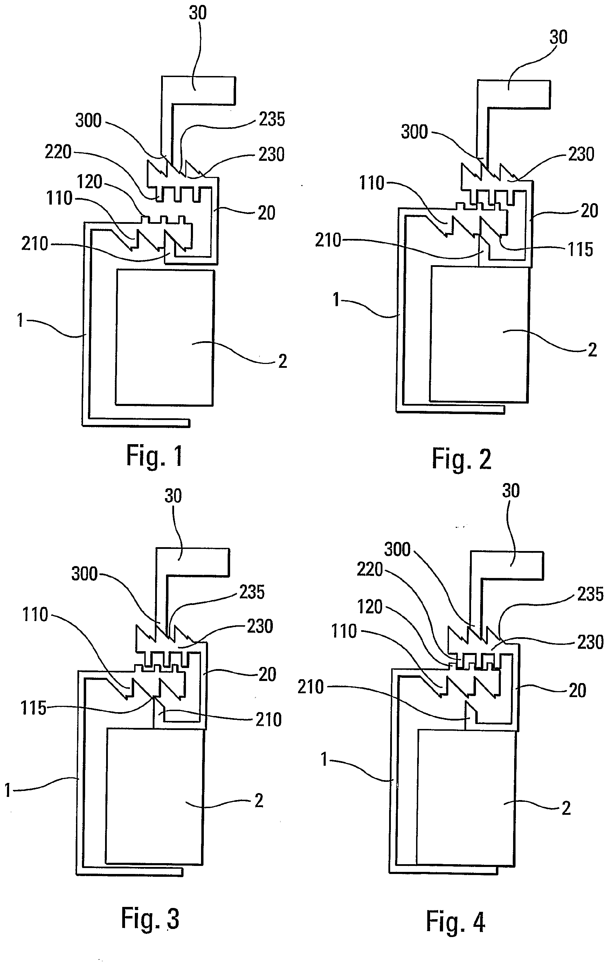

En référence aux figures 1 à 8, nous allons décrire un premier mode de réalisation de la présente invention. Selon ce premier mode de réalisation, le distributeur comporte un réservoir 2 déplaçable axialement dans un corps 1, qui sera ci-après considéré comme la partie fixe du distributeur. Le déplacement axial du réservoir 2 par rapport au corps 1 actionne l'organe de distribution et donc réalise la distribution d'une dose de produit hors dudit réservoir. Selon ce premier mode de réalisation, le distributeur comporte un organe d'actionnement 30 sur lequel l'utilisateur exerce une force d'actionnement axial pour réaliser l'actionnement du distributeur et donc pour déplacer ledit réservoir 2 par rapport au corps 1. Le distributeur comporte en outre un dispositif d'indication de doses destinées à compter ou à indiquer à chaque actionnement du distributeur la distribution d'une dose de produit fluide. L'utilisateur peut donc grâce à ce dispositif d'indication savoir combien de doses ont été distribuées hors dudit réservoir 2, ou combien de doses restent à l'intérieur dudit réservoir 2. Cette information doit être très précise, en particulier lorsque le produit fluide est un produit pharmaceutique et tout risque de sous-comptage doit être éliminé. En effet, dans l'hypothèse d'un sous-comptage, c'est-à-dire lorsque le dispositif d'indication ne compte pas une ou plusieurs distribution(s) partielle(s) ou complète(s) de dose de produit, l'utilisateur peut se retrouver avec un distributeur lui indiquant qu'il reste une ou deux dose(s) dans le réservoir, alors qu'en réalité le réservoir est vide. En cas de crise, l'utilisateur peut donc se retrouver avec un distributeur ne fonctionnant plus et ne lui permettant pas de prendre son médicament.

La présente invention permet d'éviter tout risque de sous-comptage. Pour ce faire, le dispositif d'indication de doses comporte au moins un système de sécurité, et de préférence deux. Le premier système de sécurité est destiné à garantir l'actionnement du dispositif d'indication de doses, et donc le comptage de la distribution d'une dose de produit, à partir du moment où, lors de l'actionnement du distributeur, une course partielle prédéterminée a été parcourue. Le second système de sécurité est destiné à empêcher, pendant la course de retour du distributeur, l'expulsion de dose tant que le dispositif d'indication de doses n'est pas prêt à compter la prochaine dose. En effet, la chambre de dosage du distributeur se remplie généralement lors de la phase retour du distributeur vers sa position de repos, après un actionnement précédent. Si cette course de retour n'est pas complète mais que le dispositif est actionné à nouveau avant qu'il ne revienne vers sa position de repos, il serait possible que du produit soit distribué. Or, si le dispositif d'indication de doses n'est pas revenu dans sa position de repos ou proche de celle-ci, il ne pourra pas compter cette distribution de produit. Il se produirait alors un sous-comptage. Pour éviter ceci, le second système de sécurité bloque tout nouvel actionnement, ou pour le moins toute distribution de produit. Ce blocage sera réalisé jusqu'à ce que la course de retour soit suffisante pour que le dispositif d'indication de dose puisse être de nouveau actionné et qu'il compte la prochaine dose.

En résumé, la présente invention fournit un ou deux système(s) de sécurité qui évite(nt) tout risque de sous-comptage du dispositif d'indication.

Nous allons ci-après d'abord décrire le premier système de sécurité, en référence aux figures 1 à 4.

En se référant à ces figures, on constate que le corps fixe 1 comporte un engrenage fixe 110 et que le dispositif d'indication comporte un élément de comptage 20 destiné à coopérer d'une part avec ledit engrenage fixe 110 du corps 1, et d'autre part avec l'organe d'actionnement 30 et/ou le réservoir 2. Plus particulièrement, l'élément de comptage 20 est déplaçable axialement par rapport au corps 1 ainsi qu'en rotation par rapport à celui-ci. L'élément de comptage 20 comporte un premier engrenage 210 destiné à coopérer avec ledit engrenage fixe 110 du corps et un second engrenage 230 destiné à coopérer avec un engrenage d'actionnement 300 solidaire de l'organe d'actionnement 30.

En se référant aux figures 1 et 4, il est représenté la première moitié d'un cycle d'actionnement du distributeur, c'est-à-dire le déplacement du distributeur de la position de repos (représenté sur la figure 1) vers la position d'actionnement (représenté sur la figure 4). Ainsi, lorsque l'utilisateur appuie sur l'organe d'actionnement 30 pour déplacer celui-ci axialement vers le bas sur la figure 1, le profil oblique des dents de l'engrenage d'actionnement 300 et du second engrenage 230 de l'élément de comptage sollicite l'élément de comptage 20 en déplacement axial vers le bas et aussi en rotation en raison du profil oblique des engrenages susmentionnés. Or, tant que le premier engrenage 210 de l'élément de comptage 20 coopère avec l'engrenage fixe 110 du corps 1, toute rotation de l'élément de comptage 20 est empêchée. Par conséquent, au début de la course d'actionnement du distributeur, et donc de l'organe d'actionnement 30, l'élément de comptage 20 est uniquement déplacé axialement ensemble avec l'organe d'actionnement 30, sans pouvoir tourner. Lorsque le système arrive dans la position représentée sur la figure 2, le déplacement axial de l'élément de comptage 20 a également provoqué le déplacement axial du réservoir 2 sur une première partie de course d'actionnement. Dans la position de la figure 2, le premier engrenage 210 de l'élément de comptage 20 arrive dans une position où il ne coopère plus avec l'engrenage fixe 110 du corps 1. De cette manière, l'élément de comptage, qui est sollicité en rotation par la force exercée sur l'organe d'actionnement 30, peut tourner par rapport au corps 1. A partir du moment où l'élément de comptage va un peu tourner, le dispositif d'indication de doses est actionné et le comptage d'une dose de produit est initié. Cette course partielle de déplacement axiale de l'élément de comptage 20 correspond à ladite course d'actionnement partielle prédéterminée du distributeur, plus particulièrement du réservoir 2. En effet, dans ce type de distributeur, la dose de produit n'est pas nécessairement expulsée en toute fin de course d'actionnement, mais à partir d'une course partielle prédéterminée, qui est fonction du déplacement de la soupape dans une valve, ou du déplacement du piston dans une pompe. En faisant correspondre la coopération du premier engrenage 210 de l'élément de comptage avec l'engrenage fixe 110 du corps 1 avec ladite course d'actionnement partielle prédéterminée du distributeur, on assure dès que du produit fluide risque d'être distribué hors du réservoir 2, le dispositif d'indication compte cette distribution.

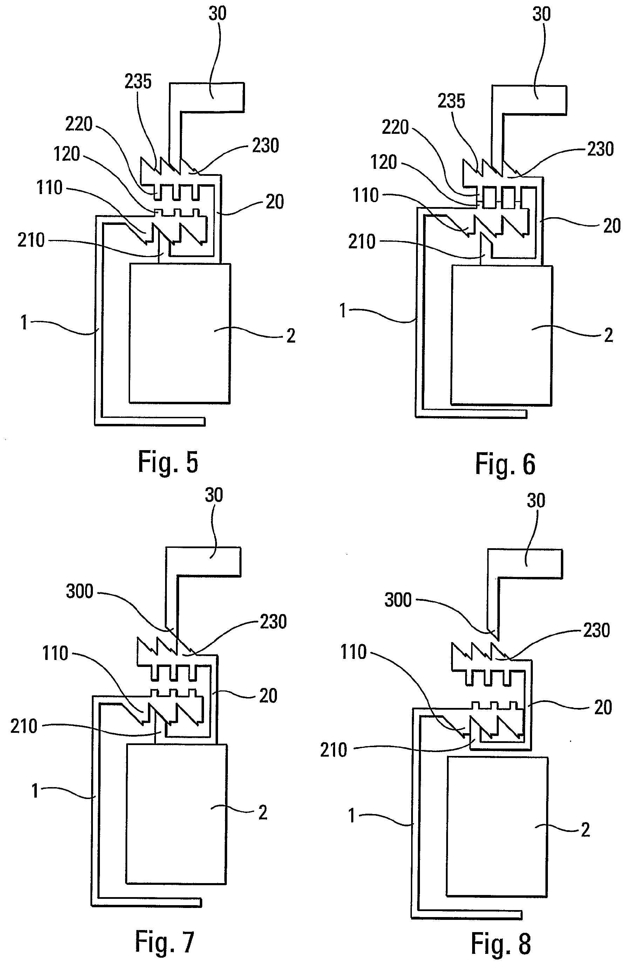

La figure 3 montre que l'engrenage fixe 110 du corps 1 comporte des moyens de butée 115, de préférence formés par une projection axiale coopérant avec le premier engrenage 210 de l'élément de comptage 20, et qui impose un nouveau déplacement axial de l'élément de comptage, très faible mais non nul, pour permettre un engrènement suffisant de l'engrenage d'actionnement 300 dans le premier palier, marqué par la butée 235 sur le second engrenage 230. Ainsi la chambre du distributeur ne pourra pas être rempli si la butée 215 n'est pas dépassée. Si elle est passée l'engrenage d'actionnement 300 se positionnera dans le second palier ou fond du second engrenage 230 de l'élément de comptage 20 avant remplissage de la chambre du distributeur pour bloquer l'expulsion de produit pharmaceutique comme il représenté sur la figure 6.

Une poursuite de la course d'actionnement amène le réservoir 2 dans la position représentée sur la figure 4, dans laquelle la course d'actionnement totale a été réalisée, et la totalité de la dose a été expulsée hors du réservoir. Toutefois, on constate que même si la force exercée par l'utilisateur cesse sur l'organe d'actionnement 30 avant la fin de la course d'actionnement totale, à partir du moment où la course d'actionnement partielle a été réalisée, le dispositif d'indication a compté la distribution de la dose, de sorte que tout sous-comptage est évité à ce niveau là.

En référence aux figures 5 à 8, on va décrire la seconde phase du cycle d'actionnement du distributeur, à savoir le retour de la position de distribution vers la position de repos (représenté sur la figure 8). En se référant à la figure 5, on constate que lorsque l'utilisateur lâche sa pression sur l'organe d'actionnement 30, le ressort de rappel (non représenté) du distributeur amène le réservoir 2 vers sa position de repos en déplaçant axialement le réservoir 2 par rapport au corps 1 dans le sens inverse à celui du déplacement d'actionnement décrit précédemment. Le déplacement du réservoir 2 provoque un déplacement axial de l'élément de comptage 20 de sorte que le premier engrenage 210 vient à nouveau coopérer avec l'engrenage fixe 110 du corps 1, mais cette fois au niveau des parties obliques, ce qui implique une rotation dudit élément de comptage, pour terminer le cycle de comptage du dispositif d'indication. Lorsque l'élément de comptage tourne par rapport au corps 1 lors de la course de retour, on constate que l'organe d'actionnement 30 vient coopérer avec le fond du second engrenage 230 de l'élément de comptage 20, alors que pendant la phase d'actionnement, l'engrenage d'actionnement 300 coopérait avec le premier palier, marqué par la butée 235, réalisée sur ledit second engrenage 230 de l'élément de comptage 20.

La figure 6 représente schématiquement une tentative de nouvel actionnement avant la fin de la course de retour du distributeur. On constate que le corps 1 comporte des projections axiales 120 qui coopèrent avec des projections axiales 220 réalisées sur l'élément de comptage 20. En variante, ces projections axiales 120, 220 peuvent être remplacée par des engrenages ayant une forme correspondante. De préférence, les profils d'extrémités axiales desdites projections 120 et 220 sont réalisées par des plans et ces projections axiales 120 et 220 sont au moins partiellement disposées face à face, de sorte que si la course de retour du distributeur est insuffisante, il est impossible d'expulser une nouvelle dose de produit, comme cela est représenté sur la figure 6. Pour pouvoir à nouveau actionner le distributeur, il faut que l'élément de comptage 20 tourne suffisamment autour de son axe de rotation pour que lesdites projections 120 et 220 soient décalées l'une par rapport à l'autre, et ainsi permettre un nouvel actionnement. Ce décalage sera réalisé en libérant l'organe d'actionnement 30 du fond du second engrenage 230 de l'élément de comptage 20 pour le positionner en face du premier palier, marqué par la butée 235, de la dent suivante. Ce nouvel actionnement peut être permis avant la fin de la course de retour totale du distributeur après que le dispositif d'indication soit de nouveau apte à compter un nouvel actionnement du distributeur. La présente invention permet de remplir cette exigence, comme le montre la figure 7 dans laquelle la course de retour n'est pas complète, mais l'organe d'actionnement 30 du distributeur et notamment l'engrenage d'actionnement 300 est dans une position où il peut coopérer avec la prochaine dent du second engrenage 230 de l'élément de comptage, de sorte qu'un nouvel actionnement à ce moment là provoque la rotation de l'élément de comptage sur la fin de son cycle précédent, ce qui amène le décalage desdites projections axiales 120 et 220, et autorisent donc un nouvel actionnement du distributeur et du dispositif d'indication de doses. Le but est de bloquer l'expulsion quand la chambre est remplie tant que le compteur n'est pas prêt à compter une prochaine dose.

Dans la figure 8, le dispositif est revenu vers sa position de repos initiale et un nouveau cycle d'actionnement peut être réalisé.

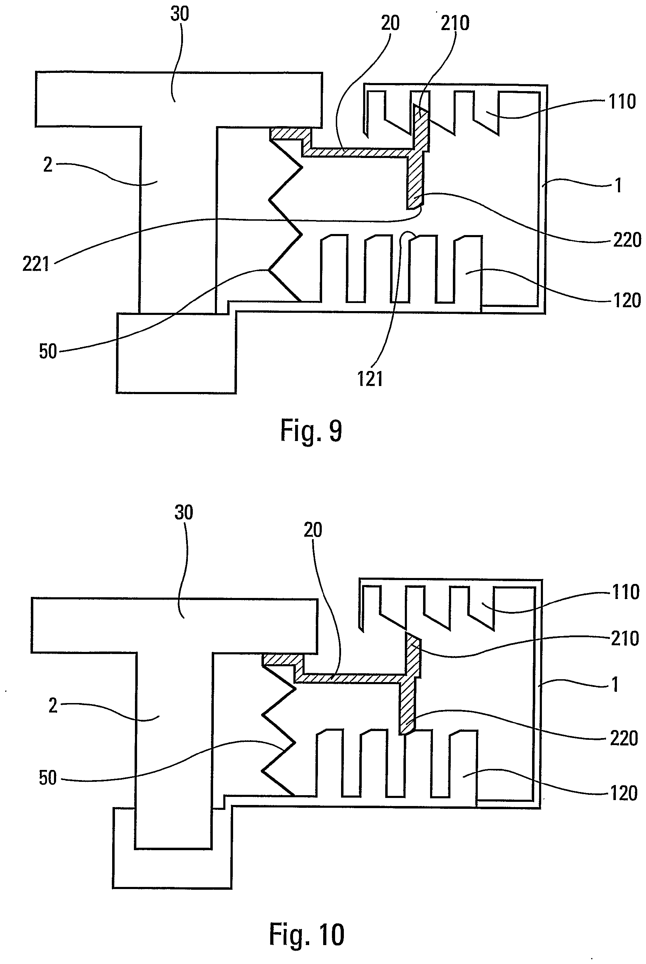

Les figures 9 à 12 représentent un deuxième mode de réalisation de l'invention. Ce deuxième mode de réalisation diffère du premier mode de réalisation par les points suivants. D'une part, le corps 1 comporte deux engrenages fixes 110 et 120 et l'élément de comptage 20 comporte deux engrenages 210, 220, chacun coopérant avec un des engrenages fixes du corps. L'organe d'actionnement 30 est solidaire du réservoir 2 et l'élément de comptage 20 est déplaçable axialement avec ledit organe d'actionnement 30. Il peut se déplacer en rotation par rapport audit organe d'actionnement 30. En fait, dans ce second mode de réalisation, il n'y a pas véritablement d'organe d'actionnement, mais l'utilisateur déplace généralement le réservoir 2 lui-même par rapport au corps 1 pour réaliser l'actionnement. Le second engrenage fixe 120 du corps 1 et le second engrenage 220 de l'élément de comptage comportent au moins partiellement une partie oblique respectivement 121 et 221, qui coopèrent l'une avec l'autre lors de l'actionnement du distributeur. Ces parties obliques sollicitent l'élément de comptage 20 à se déplacer en rotation, et donc à initier un cycle de comptage du dispositif d'indication de doses. Comme représenté sur les figures 9 et 10, lors de l'actionnement du distributeur, l'élément de comptage est d'abord déplacé axialement sans tourner en étant solidaire de l'organe d'actionnement 30 et du réservoir 2. Lorsque le distributeur a réalisé la course d'actionnement partielle prédéterminée, les parties obliques 220, 221 des seconds engrenages 120, 220 coopèrent pour faire tourner l'organe d'actionnement comme représenté sur la figure 10.

On constate que si dans la position représentée sur la figure 10, l'utilisateur arrête d'actionner le distributeur, le système revient vers sa position de repos grâce à un ressort de rappel 50 et le cycle de comptage se complète puisque le premier engrenage 210 de l'élément de comptage 20 vient coopérer avec les dents du premier engrenage fixe 110 pour solliciter encore d'avantage l'élément de comptage 20 en rotation pour l'amener à la fin de son cycle de comptage. Le premier système de sécurité est donc réalisé en ce que l'actionnement du dispositif d'indication de doses est assuré à partir du moment où le distributeur a réalisé sa course d'actionnement partielle prédéterminé à partir de laquelle une dose de produit (complète ou partielle) peut être distribuée.

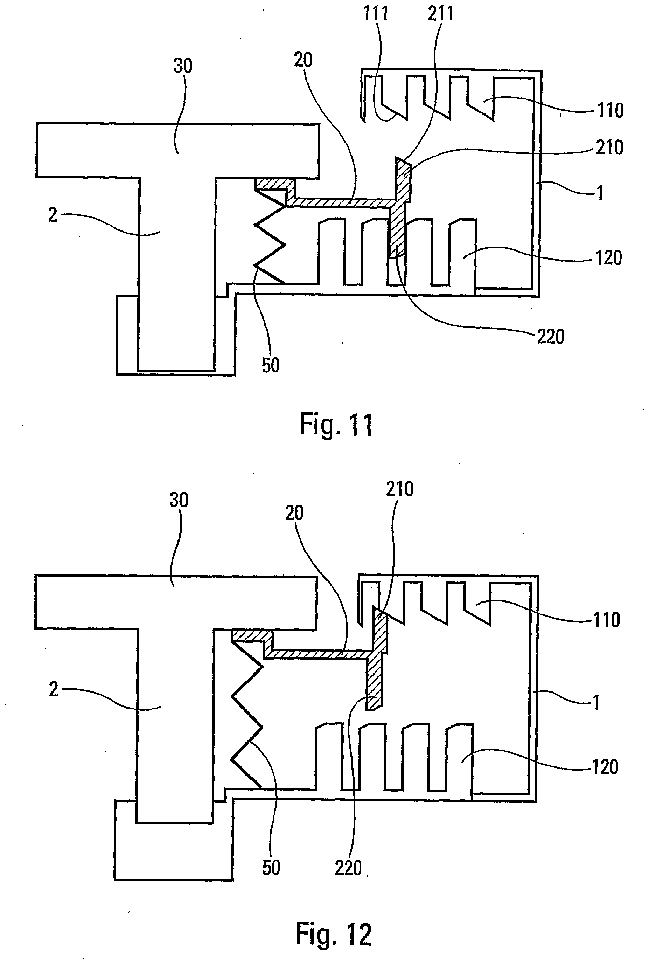

La figure 11 montre la position d'actionnement dans laquelle la course d'actionnement totale a été réalisée, et la figure 12 illustre le second système de sécurité, réalisé aux moyens du second engrenage fixe 120 et du second engrenage 220 de l'élément de comptage 20. En effet, ces deux engrenages comportent également respectivement une partie plane, ayant un profil d'extrémité axiale plan, de sorte que lorsque la course de retour du distributeur n'est pas suffisante, comme visible sur la figure 12, un nouvel actionnement du système provoque une butée axiale entre l'élément de comptage 20 et le second engrenage fixe 220 par l'intermédiaire de leur partie d'extrémité plane, ce qui empêche l'expulsion de produit pharmaceutique en bloquant l'actionnement du distributeur. Ce n'est qu'à partir du moment où une course de retour partielle prédéterminée est réalisée, que les parties obliques des seconds engrenages 120 et 220 sont face à face pour permettre un nouvel actionnement et du distributeur et du dispositif d'indication de doses.

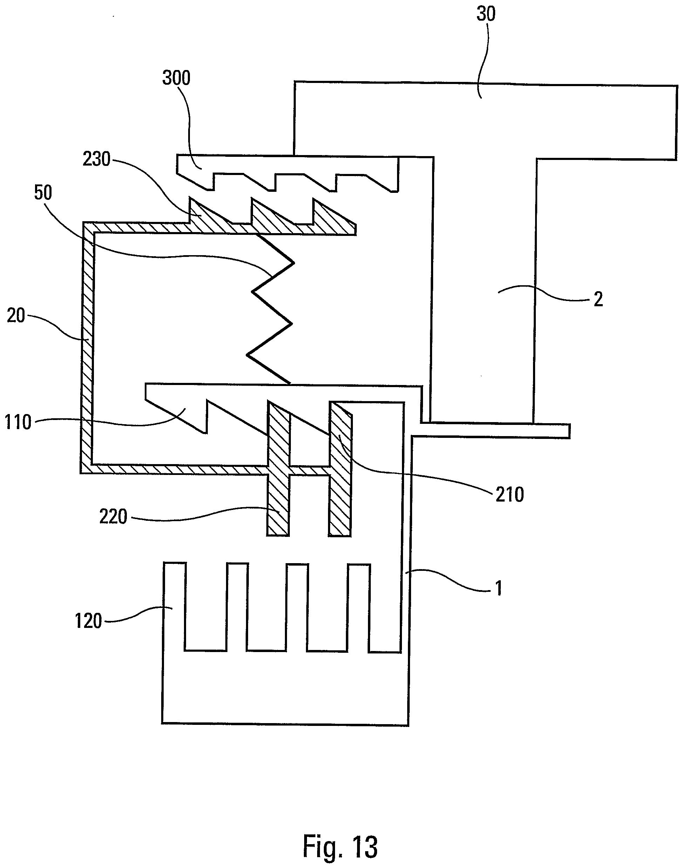

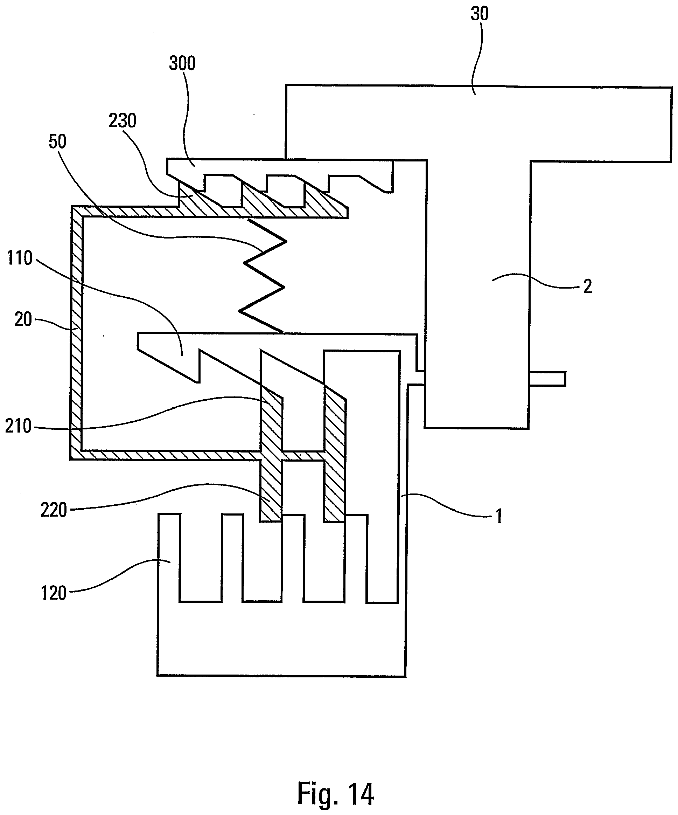

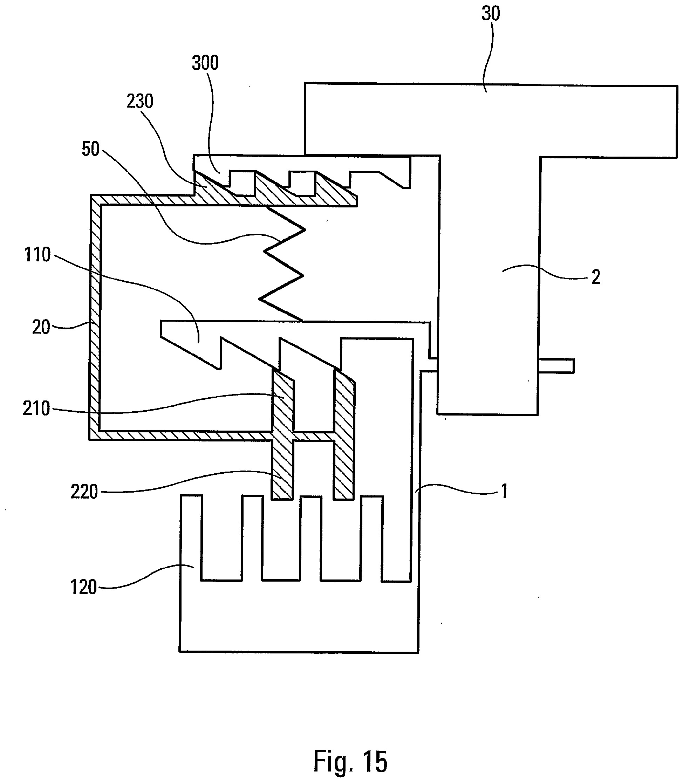

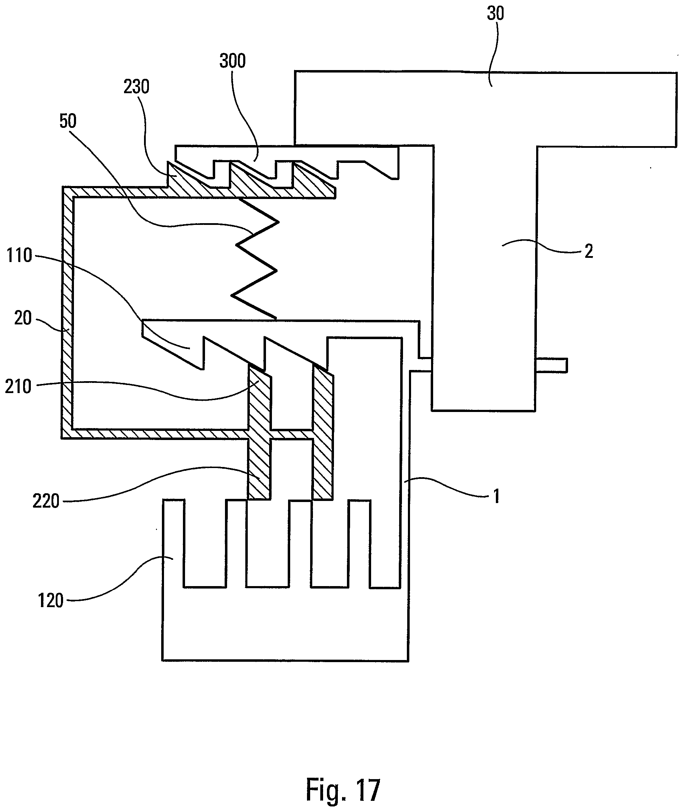

Les figures 13 à 21 montrent un troisième mode de réalisation de la présente invention. Ce troisième mode de réalisation diffère du second mode de réalisation en ce que l'élément de comptage 20 n'est pas solidaire axialement de l'organe d'actionnement 30 et du réservoir. L'organe d'actionnement 30 et le réservoir 2 comportent un engrenage d'actionnement 300 qui coopère avec une troisième engrenage 230 réalisé sur l'élément de comptage 20. Dans ce troisième mode de réalisation, la rotation de l'élément de comptage 20 lors de la course d'actionnement du distributeur n'est donc plus provoquée par le second engrenage fixe 120, mais aux moyens du troisième engrenage 230 et de l'engrenage d'actionnement 300. En faite, ce troisième mode de réalisation combine les premiers et seconds modes de réalisation décris précédemment. En début d'actionnement, lorsqu'on est dans la situation de la figure 13, la coopération entre le premier engrenage fixe 110 et le premier engrenage 210 de l'élément de comptage 20 empêche une rotation de l'élément de comptage 20 et donc provoque un déplacement axial de celui-ci. Lorsqu'on arrive dans la position représentée sur la figure 14, les premiers engrenages susmentionnés ne coopèrent plus et les profils obliques des dents de l'engrenage d'actionnement 300 et du troisième engrenage 230 de l'élément de comptage 20 provoque une rotation de l'élément de comptage 20. Ceci est réalisé à partir de ladite course d'actionnement partielle du distributeur à partir de laquelle une dose complète ou partielle de produit peut être distribuée. La figure 15 montre que si la force d'actionnement axial est supprimée à partir de ce moment là, le dispositif de comptage, grâce au ressort de rappel 50 amènera l'élément de comptage à tourner sur son cycle complet de comptage, évitant ainsi tout risque de sous-comptage.

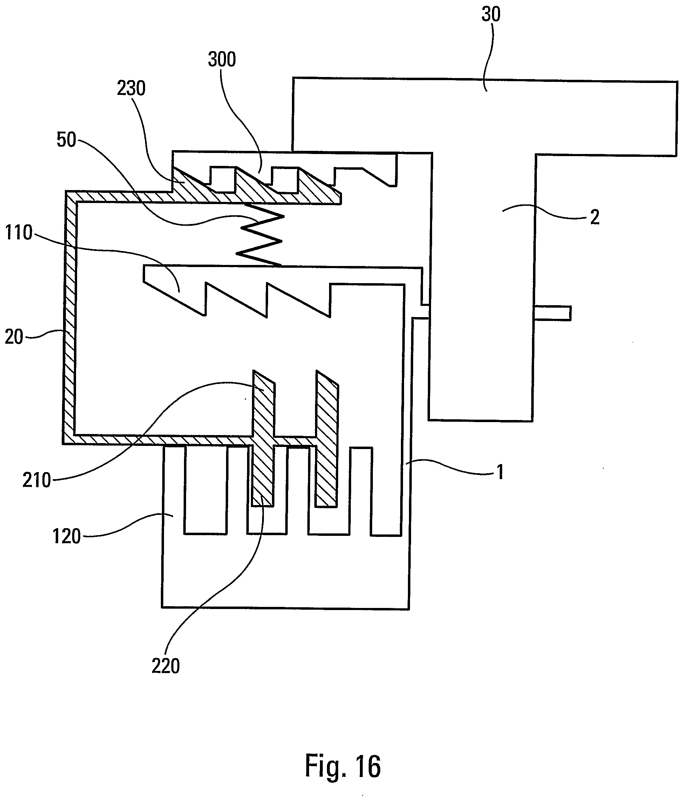

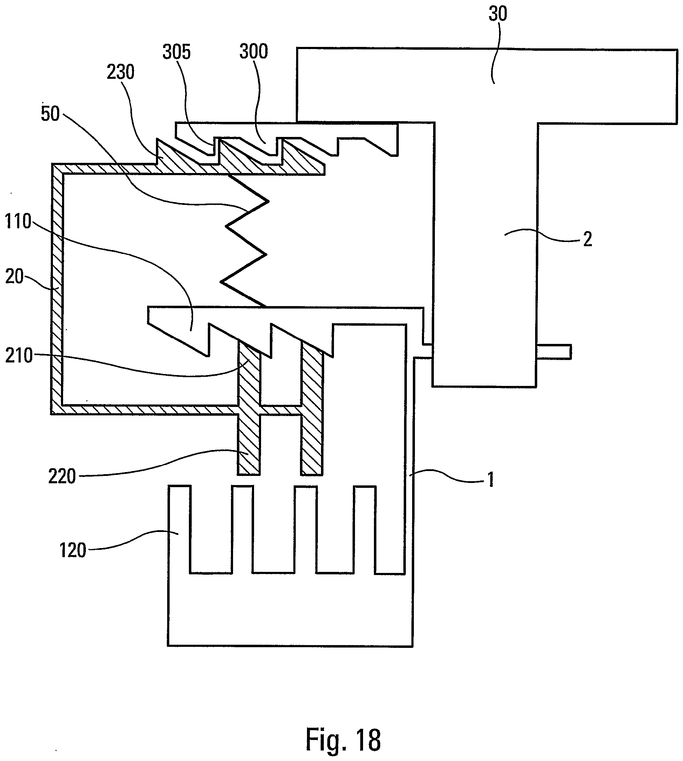

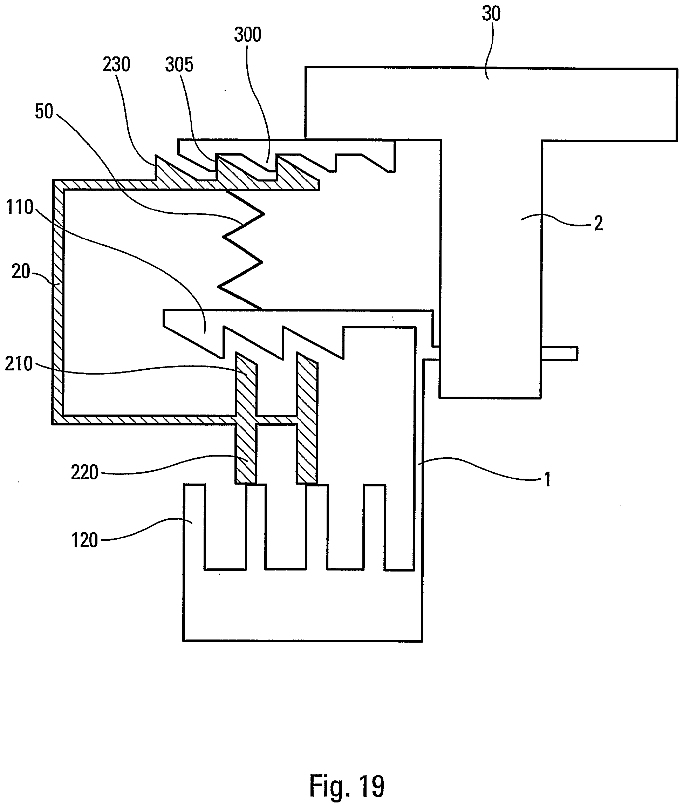

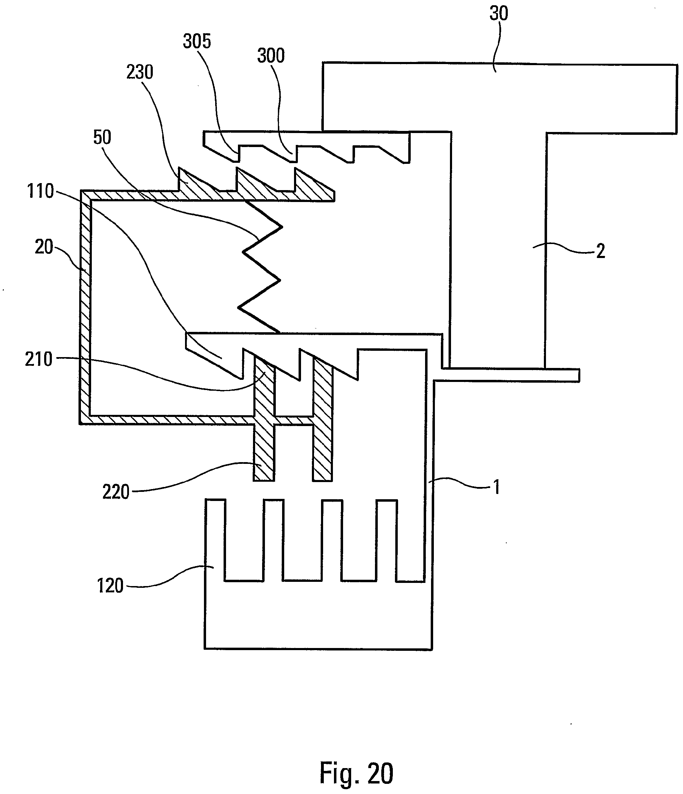

La figure 16 montre la position d'actionnement finale dans laquelle la course d'actionnement totale a été réalisée. A ce moment là, lorsque l'utilisateur relâche la force d'actionnement sur l'organe d'actionnement 30, le système remonte sous l'effet du ressort de rappel 50 et la coopération entre les premiers engrenages 210 et 110 respectivement de l'élément de comptage 20 et du corps 1 provoque la poursuite de la rotation de l'élément de comptage. Cette poursuite de la rotation est bloquée dans l'engrenage d'actionnement 300 par des moyens de butée 305. En référence à la figure 18, si l'utilisateur appuie à nouveau sur l'organe d'actionnement 30 dans cette position, on constate que l'actionnement du distributeur et du dispositif de comptage est bloqués par le fait que le deuxième engrenage 220 de l'élément de comptage et le deuxième engrenage fixe 120 du corps 1 sont face à face, au niveau de leur profil d'extrémité axiale plan. La figure 19 illustre une tentative d'actionnement alors que le second système de sécurité est opérationnel. Pour permettre un nouvel actionnement du distributeur et du dispositif d'indication de doses, il faut qu'une course de retour partielle prédéterminée soit réalisée, qui est représentée sur la figure 20. En effet, à partir de cette position, si l'utilisateur actionne à nouveau, il provoquera une rotation de l'élément de comptage 20 qui permettra un nouvel actionnement du distributeur et du dispositif d'indication.

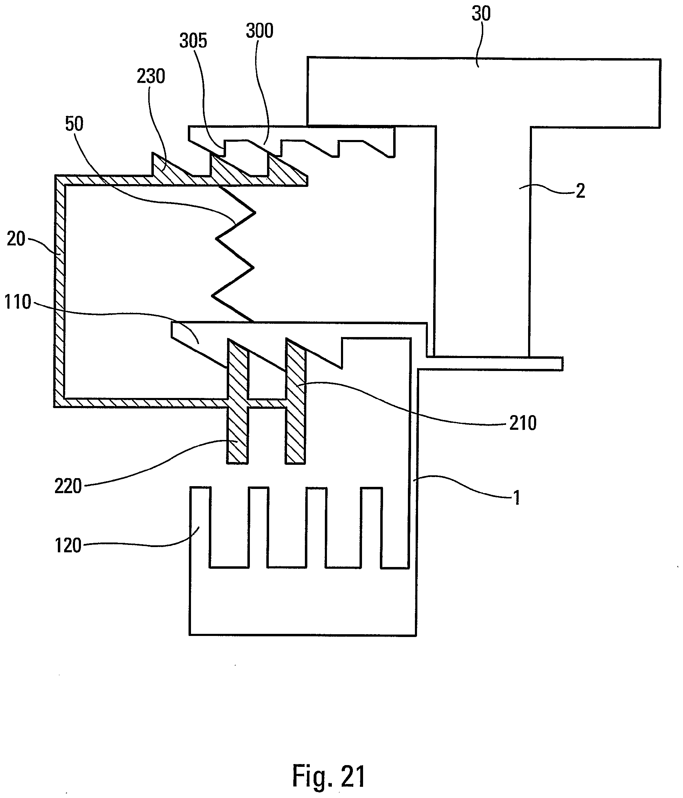

Enfin, la figure 21 montre la position dans laquelle une telle tentative d'actionnement supplémentaire est réalisée après que ladite course de retour partielle est été complétée.

Il est entendu que la description détaillée des trois modes de réalisation, donnée ci-dessus, n'est pas limitative, et que d'autres variantes de réalisation sont envisageables sans sortir du cadre de la présente invention, tel que défini par les revendications annexées.

## Revendications

1. Distributeur de produit fluide, comportant un corps (1), un réservoir de produit fluide (2), un organe de distribution, tel qu'une pompe ou une valve, monté sur ledit réservoir (2), et un dispositif d'indication de doses pour indiquer le nombre de doses de produit distribuées ou restant à distribuer à partir dudit réservoir, ledit dispositif d'indication de doses comportant un premier système de sécurité destiné à actionner le dispositif d'indication de doses à partir d'une course d'actionnement partielle prédéterminée du distributeur, même si la course d'actionnement totale n'est pas réalisée par le distributeur, ledit réservoir (2) étant déplaçable axialement par rapport audit corps (1), ledit corps (1) comportant au moins un engrenage fixe (110, 120), ledit dispositif d'indication de doses comportant un élément de comptage (20) déplaçable axialement et en rotation par rapport audit corps (1), ledit élément de comptage (20) coopérant d'une part avec ledit au moins un engrenage fixe (110, 120) dudit corps et d'autre part avec ledit réservoir (2) lorsque le distributeur est actionné, ledit corps (1) comportant un engrenage fixe (110) coopérant avec un premier engrenage (210) dudit élément de comptage (20), **caractérisé en ce que** ledit élément de comptage (20) comporte un second engrenage (230) coopérant avec un engrenage d'actionnement (300) d'un organe d'actionnement (30) du distributeur, les dents dudit second engrenage (230) et/ou dudit engrenage d'actionnement (300) étant réalisées de telle sorte qu'un déplacement axial de l'organe d'actionnement sollicite ledit élément de comptage (20) à se déplacer axialement et en rotation, l'engrenage fixe (110) empêchant une rotation dudit élément de comptage (20) jusqu'à ce que ledit élément de comptage (20) ne coopère plus avec ledit engrenage fixe (110), après un déplacement axial prédéterminé dudit élément de comptage (20) correspondant à ladite course d'actionnement partielle prédéterminée du distributeur.

2. Distributeur selon la revendication 1, dans lequel ledit dispositif d'indication de doses comporte un second système de sécurité qui, lors de la course de retour du distributeur, après distribution d'une dose, empêche la prochaine expulsion de produit jusqu'à ce que ledit distributeur a accompli une course de retour partielle prédéterminée, ledit distributeur et ledit dispositif d'indication de doses pouvant à nouveau être actionnés à partir de cette course de retour partielle prédéterminée, même si la course de retour totale n'est pas réalisée par le distributeur et que celui-ci est actionné à nouveau avant de revenir à sa position de repos.

3. Distributeur selon la revendication 1 ou 2, dans lequel l'engrenage fixe (110) comporte des moyens de butée (115) bloquant en rotation ledit élément de comptage (20) après au moins une rotation partielle dudit élément de comptage (20), un déplacement axial supplémentaire dudit élément de comptage (20) étant nécessaire pour permettre la poursuite de sa rotation et/ou le retour du dispositif d'indication vers sa position de repos.

4. Distributeur selon la revendication 3, dans lequel lesdits moyens de butée (115) comportent une projection axiale.

5. Distributeur selon l'une quelconque des revendications précédentes, dans lequel ledit engrenage fixe (110) et/ou ledit élément de comptage (20) comporte(nt) des moyens de blocage (120, 220) empêchant un nouvel actionnement du distributeur, et donc une nouvelle expulsion de produit pharmaceutique, lorsque l'élément de comptage (20) revient vers sa position de repos après un actionnement précédent, jusqu'à ce que le distributeur réalise une course de retour partielle prédéterminée à partir de laquelle le dispositif d'indication peut compter la prochaine dose.

6. Distributeur selon la revendication 5, dans lequel lesdits moyens de blocage comportent des projections axiales (120, 220) prévues respectivement sur le corps (1) et sur l'élément de comptage (20), lesdites projections (120, 220) comportant chacune un profil d'extrémité axiale plan, lesdites projections (120, 220) étant au moins partiellement face à face jusqu'à ce que l'élément de comptage (20) a accompli une rotation suffisante pour décaler lesdites projections (120, 220) correspondant à ladite course de retour partielle prédéterminée du distributeur.

7. Distributeur selon la revendication 6, dans lequel les dents du second engrenage (230) de l'élément de comptage (20) comportent un palier intermédiaire (235), l'organe d'actionnement (30) coopérant avec ledit palier (235) lors de l'actionnement du distributeur, et coopérant avec le fond dudit second engrenage (230) lors du retour vers la position de repos, après actionnement, le déplacement entre le palier intermédiaire (235) et le fond étant obtenu par rotation dudit élément de comptage (20).

8. Distributeur selon la revendication 6 ou 7, dans lequel, lorsque la course de retour partielle prédéterminée a été réalisée, l'organe d'actionnement (30) est placé face à la dent suivante du second engrenage (230) de l'élément de comptage (20), permettant un nouvel actionnement du distributeur et du dispositif d'indication de doses.

9. Distributeur selon l'une quelconque des revendications précédentes, dans lequel ledit corps (1) comporte un premier engrenage fixe (110) et un second engrenage fixe (120), ledit élément de comptage (20) comportant un premier engrenage (210) destiné à coopérer avec ledit premier engrenage fixe (110), et un second engrenage (220) destiné à coopérer avec ledit second engrenage fixe (120), ledit élément de comptage (20) étant axialement mis en contact avec l'organe d'actionnement (30) du distributeur par l'intermédiaire d'un ressort de rappel (50) et étant déplaçable en rotation par rapport audit organe d'actionnement (30), les dents dudit second engrenage (220) et second engrenage fixe (120) étant au moins partiellement obliques de telle sorte qu'un déplacement axial de l'organe d'actionnement (30) sollicite d'abord ledit élément de comptage (20) à se déplacer axialement sur une course d'actionnement partielle prédéterminée, jusqu'à ce que la partie oblique (221) dudit second engrenage (220) de l'élément de comptage (20) coopère avec ladite partie oblique (121) dudit second engrenage fixe (120), sollicitant l'élément de comptage (20) à se déplacer en rotation sur une première partie de cycle de rotation, les dents desdits premier engrenage (210) et premier engrenage fixe (110) étant au moins partiellement obliques de telle sorte que lorsque l'élément de comptage (20) revient vers sa position de repos, il est sollicité en rotation pour terminer son cycle de rotation correspondant au comptage d'un actionnement du distributeur.

10. Distributeur selon la revendication 9, dans lequel lesdits premier et second engrenages fixes (110, 120) du corps (1) et/ou lesdits premier et second engrenages (210, 220) de l'élément de comptage (20) sont décalés l'un par rapport à l'autre de telle sorte que dès que l'élément de comptage (20) est déplaçable en rotation, un retour de celui-ci vers sa position de repos sans terminer la course d'actionnement du distributeur entraîne la rotation de l'élément de comptage (20) sur son cycle complet de rotation, garantissant le comptage d'un actionnement du distributeur après ladite course d'actionnement partielle prédéterminée.

11. Distributeur selon l'une quelconque des revendications 9 ou 10, dans lequel ledit second engrenage fixe (120) du corps (1) et/ou ledit second engrenage (220) de l'élément de comptage (20) comporte(nt) des moyens de blocage empêchant un nouvel actionnement du distributeur , et donc une nouvelle expulsion de produit pharmaceutique, lorsque l'élément de comptage (20) revient vers sa position de repos après un actionnement précédent jusqu'à ce que ledit distributeur réalise une course de retour partielle prédéterminée à partir de laquelle le dispositif d'indication peut compter la prochaine dose.

12. Distributeur selon la revendication 11, dans lequel lesdits moyens de blocage comportent un profil d'extrémité axial au moins partiellement plan formé sur les dents dudit second engrenage fixe (120) du corps (1) et dudit second engrenage (220) de l'élément de comptage (20), lesdits profils plan desdites dents étant au moins partiellement face à face jusqu'à ce que l'élément de comptage (20) a accompli une rotation suffisante pour décaler lesdites dents, correspondant à ladite course de retour partielle prédéterminée du distributeur.

13. Distributeur selon l'une quelconque des revendications 9 à 12, dans lequel ledit organe d'actionnement (30) du distributeur est solidaire dudit réservoir (2) et se déplace axialement avec lui.

14. Distributeur selon l'une quelconque des revendications précédentes, dans lequel ledit corps (1) comporte un premier engrenage fixe (110) et un second engrenage fixe (120), ledit élément de comptage (20) comportant un premier engrenage (210) destiné à coopérer avec ledit premier engrenage fixe (110), un second engrenage (220) destiné à coopérer avec ledit second engrenage fixe (120), et un troisième engrenage (230) destiné à coopérer avec un engrenage d'actionnement (300), solidaire d'un organe d'actionnement (30) du distributeur, les dents dudit troisième engrenage (230) et/ou dudit engrenage d'actionnement (300) étant réalisées de tel sorte qu'un déplacement axial de l'organe d'actionnement (30) sollicite ledit élément de comptage (20) à se déplacer axialement et en rotation, le premier engrenage fixe (110) empêchant une rotation dudit élément de comptage (20) jusqu'à ce que ledit élément de comptage (20) ne coopère plus avec ledit premier engrenage fixe (110), après un déplacement axial prédéterminé dudit élément de comptage (20) correspondant à ladite course d'actionnement partielle prédéterminée du distributeur.

15. Distributeur selon la revendication 14, dans lequel ledit second engrenage fixe (120) du corps (1) et/ou ledit second engrenage (220) de l'élément de comptage (20) comporte(nt) des moyens de blocage empêchant un nouvel actionnement du distributeur, et donc une nouvelle expulsion de produit pharmaceutique, lorsque l'élément de comptage (20) revient vers sa position de repos après un actionnement précédent jusqu'à ce que ledit distributeur réalise une course de retour partielle prédéterminée à partir de laquelle le dispositif d'indication peut compter la prochaine dose.

16. Distributeur selon la revendication 15, dans lequel lesdits moyens de blocage comportent un profil d'extrémité axial plan formé sur les dents dudit second engrenage fixe (120) et dudit second engrenage (220), lesdites dents étant au moins partiellement face à face jusqu'à ce que l'élément de comptage (20) a accompli une rotation suffisante pour décaler lesdites dents, correspondant à ladite course de retour partielle prédéterminée du distributeur.

17. Distributeur selon la revendication 15 ou 16, dans lequel ledit engrenage d'actionnement (300) comporte des moyens de butée (305) limitant la rotation de l'élément de comptage (20) jusqu'à ce que l'organe d'actionnement (30) a réalisé ladite course de retour partielle prédéterminée.

18. Distributeur selon la revendication 17, dans lequel lesdits moyens de butée (305) comportent une projection axiale formée sur ledit engrenage d'actionnement (300).

19. Distributeur selon l'une quelconque des revendications 14 à 18, dans lequel ledit organe d'actionnement (30) du distributeur est solidaire dudit réservoir (2) et se déplace axialement avec lui.

## Patentansprüche

1. Spender für ein fluides Produkt, umfassend einen Körper (1), einen Behälter für fluides Produkt (2), eine Ausgabeeinrichtung, wie eine Pumpe oder ein Ventil, die auf dem Behälter (2) montiert ist, und eine Dosisanzeigevorrichtung, um die Anzahl an Dosen des aus dem Behälter ausgegebenen oder auszugebenden Produkts anzuzeigen, wobei die Dosisanzeigevorrichtung Folgendes umfasst:
ein erstes Sicherheitssystem, das dazu bestimmt ist, die Dosisanzeigevorrichtung durch einen vorbestimmten, teilweisen Betätigungshub des Spenders zu betätigen, auch wenn nicht der gesamte Betätigungshub von dem Spender ausgeführt wird, wobei der Behälter (2) in Bezug auf den Körper (1) axial verschiebbar ist, wobei der Körper (1) mindestens ein feststehendes Räderwerk (110, 120) umfasst, wobei die Dosisanzeigevorrichtung ein Zählelement (20) umfasst, das axial und drehbar in Bezug auf den Körper (1) verschiebbar ist, wobei das Zählelement (20) einerseits mit dem mindestens einen feststehenden Räderwerk (110, 120) des Körpers und andererseits mit dem Behälter (2) zusammenwirkt, wenn der Spender betätigt wird, wobei der Körper (1) ein feststehendes Räderwerk (110) umfasst, das mit einem ersten Räderwerk (210) des Zählelements (20) zusammenwirkt, **dadurch gekennzeichnet, dass** das Zählelement (20) ein zweites Räderwerk (230) umfasst, das mit einem Betätigungsräderwerk (300) einer Betätigungseinrichtung (30) des Spenders zusammenwirkt, wobei die Zähne des zweiten Räderwerks (230) und/oder des Betätigungsräderwerks (300) derart ausgeführt sind, dass eine axiale Verschiebung der Betätigungseinrichtung das Zählelement (20) so belastet, dass es sich axial und drehend verschiebt, wobei das feststehende Räderwerk (110) eine Drehung des Zählelements (20) verhindert, bis das Zählelement (20) nach einer vorbestimmten axialen Verschiebung des Zählelements (20) entsprechend dem vorbestimmten teilweisen Betätigungshub des Spenders nicht mehr mit dem feststehenden Räderwerk (110) zusammenwirkt.

2. Spender nach Anspruch 1, wobei die Dosisanzeigevorrichtung ein zweites Sicherheitssystem umfasst, welches beim Rückhub des Spenders nach der Ausgabe einer Dosis die nächste Austreibung des Produkts verhindert, bis der Spender einen vorbestimmten teilweisen Rückhub ausgeführt hat, wobei der Spender und die Dosisanzeigevorrichtung durch diesen vorbestimmten teilweisen Rückhub erneut betätigt werden können, auch wenn nicht der gesamte Rückhub von dem Spender ausgeführt wurde und dieser vor seiner Rückkehr in seine Ruheposition erneut betätigt wird.

3. Spender nach Anspruch 1 oder 2, wobei das feststehende Räderwerk (110) Anschlagmittel (115) umfasst, die das Zählelement (20) nach zumindest einer teilweisen Drehung des Zählelements (20) in seiner Drehung blockieren, wobei eine zusätzliche axiale Verschiebung des Zählelements (20) notwendig ist, um die Fortführung seiner Drehung und/oder die Rückkehr der Anzeigevorrichtung in ihre Ruheposition zu ermöglichen.

4. Spender nach Anspruch 3, wobei die Anschlagmittel (115) einen axialen Vorsprung umfassen.

5. Spender nach einem der vorhergehenden Ansprüche, wobei das feststehende Räderwerk (110) und/oder das Zählelement (20) Sperrmittel (120, 220) umfasst (umfassen), die eine erneute Betätigung des Spenders, und somit eine erneute Austreibung von pharmazeutischem Produkt, verhindern, wenn das Zählelement (20) nach einer vorausgehenden Betätigung in seine Ruheposition zurückkehrt, bis der Spender einen vorbestimmten teilweisen Rückhub ausübt, ab welchem die Anzeigevorrichtung die nächste Dosis zählen kann.

6. Spender nach Anspruch 5, wobei die Sperrmittel axiale Vorsprünge (120, 220) umfassen, die jeweils auf dem Körper (1) und auf dem Zählelement (20) vorgesehen sind, wobei die Vorsprünge (120, 220) jeweils ein axiales, ebenes Endprofil umfassen, wobei sich die Vorsprünge (120, 220) zumindest teilweise gegenüberliegen, bis das Zählelement (20) eine ausreichende Drehung ausgeführt hat, um die Vorsprünge (120, 220) entsprechend dem vorbestimmten teilweisen Rückhub des Spenders zu verschieben.

7. Spender nach Anspruch 6, wobei die Zähne des zweiten feststehenden Räderwerks (230) des Zählelements (20) ein Zwischenlager (235) umfassen, wobei die Betätigungseinrichtung (30) während der Betätigung des Spenders mit dem Lager (235) zusammenwirkt und während der Rückkehr in die Ruheposition, nach der Betätigung, mit dem Boden des zweiten Räderwerks (230) zusammenwirkt, wobei die Verschiebung zwischen dem Zwischenlager (235) und dem Boden durch Drehung des Zählelements (20) erhalten wird.

8. Spender nach Anspruch 6 oder 7, wobei, wenn der vorbestimmte teilweise Rückhub ausgeführt wurde, die Betätigungseinrichtung (30) dem folgenden Zahn des zweiten Räderwerks (230) des Zählelements (20) gegenüberliegend angeordnet wird, was eine erneute Betätigung des Spenders und der Dosisanzeigevorrichtung ermöglicht.

9. Spender nach einem der vorhergehenden Ansprüche, wobei der Körper (1) ein erstes feststehendes Räderwerk (110) und ein zweites feststehendes Räderwerk (120) umfasst, wobei das Zählelement (20) ein erstes Räderwerk (210), das dazu bestimmt ist, mit dem ersten feststehenden Räderwerk (110) zusammenzuwirken, und ein zweites Räderwerk (220), das dazu bestimmt ist, mit dem zweiten feststehenden Räderwerk (120) zusammenzuwirken, umfasst, wobei das Zählelement (20) mittels einer Spannfeder (50) axial mit der Betätigungseinrichtung (30) des Spenders in Kontakt gebracht wird und drehend in Bezug auf die Betätigungseinrichtung (30) verschiebbar ist, wobei die Zähne des zweiten Räderwerks (220) und des zweiten feststehenden Räderwerks (120) zumindest teilweise derart schräg sind, dass eine axiale Verschiebung der Betätigungseinrichtung (30) zunächst das Zählelement (20) so belastet, dass es sich axial auf einem vorbestimmten teilweisen Betätigungshub verschiebt, bis der schräge Teil (221) des zweiten Räderwerks (220) des Zählelements (20) mit dem schrägen Teil (121) des zweiten feststehenden Räderwerks (120) zusammenwirkt und dabei das Zählelement (20) so belastet, dass es sich drehend auf einem ersten Drehzyklusteil verschiebt, wobei die Zähne des ersten Räderwerks (210) und des ersten feststehenden Räderwerks (110) zumindest teilweise derart schräg sind, dass, wenn das Zählelement (20) in seine Ruheposition zurückkehrt, dieses drehend belastet wird, um seinen Drehzyklus entsprechend dem Zählen einer Betätigung des Spenders zu beenden.

10. Spender nach Anspruch 9, wobei das erste und das zweite feststehende Räderwerk (110, 120) des Körpers (1) und/oder das erste und das zweite Räderwerk (210, 220) des Zählelements (20) derart zueinander versetzt sind, dass, sobald das Zählelement (20) drehend verschiebbar ist, eine Rückkehr von diesem in seine Ruheposition, ohne den Betätigungshub des Spenders zu beenden, die Drehung des Zählelements (20) auf seinem gesamten Drehzyklus mit sich bringt, was das Zählen einer Betätigung des Spenders nach dem vorbestimmten teilweisen Betätigungshub gewährleistet.

11. Spender nach einem der Ansprüche 9 oder 10, wobei das zweite feststehende Räderwerk (120) des Körpers (1) und/oder das zweite Räderwerk (220) des Zählelements (20) Sperrmittel umfasst (umfassen), die eine erneute Betätigung des Spenders und somit eine erneute Austreibung von pharmazeutischem Produkt verhindern, wenn das Zählelement (20) nach einer vorausgehenden Betätigung in seine Ruheposition zurückkehrt, bis der Spender einen vorbestimmten teilweisen Rückhub ausübt, ab welchem die Anzeigevorrichtung die nächste Dosis zählen kann.

12. Spender nach Anspruch 11, wobei die Sperrmittel ein zumindest teilweise ebenes axiales Endprofil umfassen, das auf den Zähnen des zweiten feststehenden Räderwerks (120) des Körpers (1) und des zweiten Räderwerks (220) des Zählelements (20) gebildet ist, wobei die ebenen Profile der Zähne sich zumindest teilweise gegenüberliegen, bis das Zählelement (20) eine ausreichende Drehung ausgeführt hat, um die Zähne entsprechend dem vorbestimmten teilweisen Rückhub des Spenders zu verschieben.

13. Spender nach einem der Ansprüche 9 bis 12, wobei die Betätigungseinrichtung (30) des Spenders mit dem Behälter (2) einstückig ausgebildet ist und sich axial mit diesem verschiebt.

14. Spender nach einem der vorhergehenden Ansprüche, wobei der Körper (1) ein erstes feststehendes Räderwerk (110) und ein zweites feststehendes Räderwerk (120) umfasst, wobei das Zählelement (20) ein erstes Räderwerk (210), das dazu bestimmt ist, mit dem ersten feststehenden Räderwerk (110) zusammenzuwirken, ein zweites Räderwerk (220), das dazu bestimmt ist, mit dem zweiten feststehenden Räderwerk (120) zusammenzuwirken, und ein drittes Räderwerk (230) umfasst, das dazu bestimmt ist, mit einem Betätigungsräderwerk (300), das mit einer Betätigungseinrichtung (30) des Spenders einstückig ausgebildet ist, zusammenzuwirken, wobei die Zähne des dritten Räderwerks (230) und/oder des Betätigungsräderwerks (300) derart ausgeführt sind, dass eine axiale Verschiebung der Betätigungseinrichtung (30) das Zählelement (20) so belastet, dass es sich axial und drehend verschiebt, wobei das erste feststehende Räderwerk (110) eine Drehung des Zählelements (20) verhindert, bis das Zählelement (20) nach einer vorbestimmten axialen Verschiebung des Zählelements (20) entsprechend dem vorbestimmten teilweisen Betätigungshub des Spenders nicht mehr mit dem ersten feststehenden Räderwerk (110) zusammenwirkt.

15. Spender nach Anspruch 14, wobei das zweite feststehende Räderwerk (120) des Körpers (1) und/oder das zweite Räderwerk (220) des Zählelements (20) Sperrmittel umfasst (umfassen), die eine erneute Betätigung des Spenders und somit eine erneute Austreibung von pharmazeutischem Produkt verhindern, wenn das Zählelement (20) nach einer vorausgehenden Betätigung in seine Ruheposition zurückkehrt, bis der Spender einen vorbestimmten teilweisen Rückhub ausübt, ab welchem die Anzeigevorrichtung die nächste Dosis zählen kann.

16. Spender nach Anspruch 15, wobei die Sperrmittel ein ebenes axiales Endprofil umfassen, das auf den Zähnen des zweiten feststehenden Räderwerks (120) und des zweiten Räderwerks (220) ausgebildet ist, wobei sich die Zähne zumindest teilweise gegenüberliegen, bis das Zählelement (20) eine ausreichende Drehung ausgeführt hat, um die Zähne entsprechend dem vorbestimmten teilweisen Rückhub des Spenders zu verschieben.

17. Spender nach Anspruch 15 oder 16, wobei das Betätigungsräderwerk (300) Anschlagmittel (305) umfasst, die die Drehung des Zählelements (20) verhindern, bis die Betätigungseinrichtung (30) den vorbestimmten teilweisen Rückhub ausgeführt hat.

18. Spender nach Anspruch 17, wobei die Anschlagmittel (305) einen axialen Vorsprung umfassen, der auf dem Betätigungsräderwerk (300) ausgebildet ist.

19. Spender nach einem der Ansprüche 14 bis 18, wobei die Betätigungseinrichtung (30) des Spenders mit dem Behälter (2) einstückig ausgebildet ist und sich axial mit diesem verschiebt.

## Claims

1. A fluid dispenser, comprising a body (1), a fluid reservoir (2), a dispenser member, such as a pump or a valve, mounted on said reservoir (2), and a dose indicator device for indicating the number of doses of fluid that have been dispensed or that remain to be dispensed from said reservoir, said dose indicator device including a first safety system for actuating the dose indicator device once the dispenser has performed a predetermined incomplete actuation stroke, even if the dispenser does not perform the complete actuation stroke, said reservoir (2) being axially displaceable relative to said body (1), said body (1) including at least one stationary gear (110, 120), said dose indicator device including a counter element (20) that is displaceable axially and in rotation relative to said body (1), said counter element (20) co-operating while the dispenser is being actuated firstly with said at least one stationary gear (110, 120) of said body and secondly with said reservoir (2), said body (1) includes a stationary gear (110) co-operating with a first gear (210) of said counter element (20), **characterized in that** said counter element (20) includes a second gear (230) co-operating with an actuator gear (300) of an actuator member (30) of the dispenser, the teeth of said second gear (230) and/or of said actuator gear (300) being made so that axial displacement of the actuator member causes said counter element (20) to be displaced axially and in rotation, the stationary gear (110) preventing said counter element (20) from turning until said counter element (20) no longer co-operates with said stationary gear (110), after a predetermined axial displacement of said counter element (20) corresponding to said predetermined incomplete actuation stroke of the dispenser.

2. A dispenser according to claim 1, in which said dose indicator device includes a second safety system which, during the return stroke of the dispenser, after dispensing a dose, prevents the fluid from being expelled again until said dispenser has completed a predetermined incomplete return stroke, said dispenser and said dose indicator device being capable of being actuated once again, once the predetermined incomplete return stroke has been performed, even if the dispenser does not perform the complete return stroke and said dispenser is actuated once again before returning to it rest position.

3. A dispenser according to claim 1 or 2, in which the stationary gear (110) includes abutment means (115) preventing said counter element (20) from turning once said counter element (20) has turned at least in part, an additional axial displacement of said counter element (20) being necessary to enable it to continue to turn and/or to return the indicator device to its rest position.

4. A dispenser according to claim 3, in which said abutment means (115) comprise an axial projection.

5. A dispenser according to any preceding claim, in which said stationary gear (110) and/or said counter element (20) include(s) blocking means (120, 220) preventing the dispenser from being actuated once again, and thus preventing any pharmaceutical from being expelled once again, while the counter element (20) is returning to its rest position after a preceding actuation, and until the dispenser has completed a predetermined incomplete return stroke after which the indicator device can count the next dose.

6. A dispenser according to claim 5, in which said blocking means comprise axial projections (120, 220) provided on the body (1) and on the counter element (20) respectively, each of said projections (120, 220) having an axial end-profile that is plane, said projections (120, 220) facing each other at least in part, until the counter element has turned sufficiently to offset said projections (120, 220) with that sufficient turn corresponding to said predetermined incomplete return stroke of the dispenser.

7. A dispenser according to claim 6, in which the teeth of the second gear (230) of the counter element (20) include an intermediate step (235), the actuator member (30) co-operating with said step (235) while the dispenser is being actuated, and co-operating with the end wall of said second gear (230) while returning to the rest position, after being actuated, the displacement between the intermediate step (235) and the end wall being obtained by said counter element (20) turning.

8. A dispenser according to claim 6 or claim 7, in which, once the predetermined incomplete return stroke has been performed, the actuator member (30) is positioned facing the following tooth of the second gear (230) of the counter element (20), enabling the dispenser and the dose indicator device to be actuated once again.

9. A dispenser according to any preceding claim, in which said body (1) includes a first stationary gear (110), and a second stationary gear (120), said counter element (20) including a first gear (210) for co-operating with said first stationary gear (110) and a second gear (220) for co-operating with said second stationary gear (120), said counter element (20) being put axially into contact with the actuator member (30) of the dispenser by means of a return spring (50) and being turnable relative to said actuator member (30), the teeth of said second gear (220) and of said second stationary gear (120) being oblique, at least in part, so that an axial displacement of the actuator member (30) initially causes said counter element (20) to be displaced axially over a predetermined incomplete actuation stroke, until the oblique portion (221) of said second gear (220) of the counter element (20) co-operates with said oblique portion (121) of said second stationary gear (120), causing the counter element (20) to be turned over a first portion of a turn cycle, the teeth of said first gear (210) and of said first stationary gear (110) being oblique at least in part so that when the counter element (20) returns to its rest position, it is caused to turn so as to terminate its turn cycle which corresponds to one actuation of the dispenser being counted.

10. A dispenser according to claim 9, in which said first and second stationary gears (110, 120) of the body (1) and/or said first and second gears (210, 220) of the counter element (20) are offset relative to each other so that whenever the counter element (20) is displaceable in rotation, returning said counter element to its rest position without terminating the actuation stroke of the dispenser causes the counter element (20) to turn over its complete turn cycle, guaranteeing that one actuation of the dispenser is counted after said predetermined incomplete actuation stroke.

11. A dispenser according to claim 9 or claim 10, in which said second stationary gear (120) of the body (1) and/or said second gear (220) of the counter element (20) include(s) blocking means preventing the dispenser from being actuated once again, and thus preventing pharmaceutical from being expelled once again, while the counter element (20) is returning to its rest position following a preceding actuation, and until said dispenser has performed a predetermined incomplete return stroke after which the indicator device can count the next dose.

12. A dispenser according to claim 11, in which said blocking means have an axial end-profile that is plane, at least in part, and that is formed on the teeth of said second stationary gear (120) of the body (1) and on the teeth of said second gear (220) of the counter element (20), said plane profiles of said teeth facing each other, at least in part, until the counter element (20) has completed a turn that is sufficient to offset said teeth, with that sufficient turn corresponding to said predetermined incomplete return stroke of the dispenser.

13. A dispenser according to any one of claims 9 to 12, in which said actuator member (30) of the dispenser is secured to said reservoir (2) and is displaced axially therewith.

14. A dispenser according to any preceding claim, in which said body (1) includes a first stationary gear (110) and a second stationary gear (120), said counter element (20) comprising a first gear (210) for co-operating with said first stationary gear (110), a second gear (220) for co-operating with said second stationary gear (120), and a third gear (230) for co-operating with an actuator gear (300), secured to an actuator member (30) of the dispenser, the teeth of said third gear (230) and/or of said actuator gear (300) being made so that axial displacement of the actuator member (30) causes said counter element (20) to be displaced axially and in rotation, the first stationary gear (110) preventing said counter element (20) from turning until said counter element (20) no longer co-operates with said stationary gear (110), after a predetermined axial displacement of said counter element (20) corresponding to said predetermined incomplete actuation stroke of the dispenser.

15. A dispenser according to claim 14, in which said second stationary gear (120) of the body (1) and/or said second gear (220) of the counter element (20) include(s) blocking means preventing the dispenser from being actuated once again, and thus preventing any pharmaceutical from being expelled once again, while the counter element (20) is returning to its rest position following a preceding actuation until said dispenser has performed a predetermined incomplete return stroke after which the indicator device can count the next dose.

16. A dispenser according to claim 15, in which said blocking means have an axial end-profile that is plane and that is formed on the teeth of said second stationary gear (120) and on the teeth of said second gear (220), said teeth facing each other at least in part, until the counter element (20) has completed a turn that is sufficient to offset said teeth, with that sufficient turn corresponding to said predetermined incomplete return stroke of the dispenser.

17. A dispenser according to claim 15 or claim 16, in which said actuator gear (300) includes abutment means (305) limiting the extent to which the counter element (20) can turn until the actuator member (30) has performed said predetermined incomplete return stroke.

18. A dispenser according to claim 17, in which said abutment means (305) comprise an axial projection formed on said actuator gear (300).

19. A dispenser according to any one of claims 14 to 18 in which said actuator member (30) of the dispenser is secured to said reservoir (2) and is displaced axially therewith.
